(19) Europäisches Patentamt
European Patent Office
Office européen des brevets

(11) Veröffentlichungsnummer: **0 136 662**
**B1**

(12) # EUROPÄISCHE PATENTSCHRIFT

(45) Veröffentlichungstag der Patentschrift: **25.11.87**

(51) Int. Cl.⁴: **C 07 D 493/08** // (C07D493/08, 319:00, 311:00)

(21) Anmeldenummer: **84111551.2**

(22) Anmeldetag: **27.09.84**

(54) **2,6-Dioxa-Bicyclo- 2,2,2 -octan-7-yl-acetaldehyde.**

(30) Priorität: **01.10.83 DE 3335827**

(43) Veröffentlichungstag der Anmeldung: **10.04.85 Patentblatt 85/15**

(45) Bekanntmachung des Hinweises auf die Patenterteilung: **25.11.87 Patentblatt 87/48**

(84) Benannte Vertragsstaaten: **AT BE CH DE FR GB IT LI NL SE**

(56) Entgegenhaltungen: **DE-A-2 129 507** **FR-A-2 389 628**

(73) Patentinhaber: **Kali- Chemie Pharma GmbH, Hans-Böckler- Allee 20 Postfach 220, D-3000 Hannover 1 (DE)**

(72) Erfinder: **Thies, Peter Willibrord Dipl.- Chem. Dr.phil., Ihmeplatz 6, D-3000 Hannover 1 (DE)**
Erfinder: **David, Samuel Dipl.- Chem. Dr. rer. nat., Gollstrasse 9, D-3000 Hannover 73 (DE)**

(74) Vertreter: **Lauer, Dieter, Dr., c/o Kali- Chemie AG Postfach 220 Hans- Böckler- Allee 20, D-3000 Hannover 1 (DE)**

EP 0 136 662 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft 2,6-Dioxa-bicyclo-[2,2,2]-octan-7-yl-acetaldehyde und deren Herstellung.

Der Erfindung liegt die Aufgabe zugrunde, neue Zwischenprodukte zu finden zur Herstellung neuer in 5-Stellung basisch substituierter 2,10-Dioxa-tricyclo-[5,3,1,$0^{3,8}$]-undecan-Derivate mit wertvollen pharmakologischen Eigenschaften, insbesondere neuer N-(2,10-Dioxatricyclo-[5,3,1,$0^{3,8}$]-undecan-5-yl)-tryptamin-Derivate, welche Gegenstand der Parallelanmeldung EP-A-136 661 sind und wertvolle pharmakologische Eigenschaften, insbesondere Herzkreislauf-Wirkungen, z. B. blutdrucksenkende Wirkung besitzen.

Gegenstand der Erfindung sind neue Verbindungen der allgemeinen Formel (I)

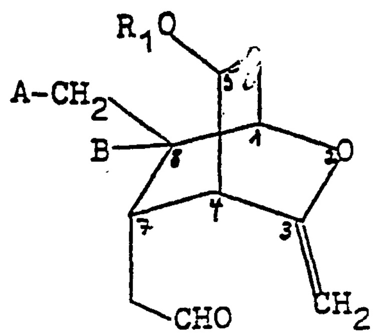

I

worin $R_1$ Alkyl mit 1-4 Kohlenstoffatomen oder Benzyl bedeutet und A und B je Wasserstoff bedeuten oder gemeinsam eine Bindung bilden, sowie ein Verfahren zu deren Herstellung.

Vorzugsweise stellt $R_1$ in den Verbindungen der Formel (I) Methyl dar.

In den Verbindungen der Formel (I) können die Substituenten an den Zentren C5 und C8 jeweils in R- oder S-Stellung angeordnet sein, so daß die Verbindungen der Formel (I) in mehreren diastereomeren Formen auftreten können. Falls A und B je Wasserstoff bedeuten, ist C8 bevorzugt R-konfiguriert. C5 ist bevorzugt R-konfiguriert.

Die vorliegende Erfindung umfaßt alle diastereoisomeren Formen der Verbindungen der Formel (I).

Die Verbindungen der Formel (I) werden erfindungsgemäß erhalten, indem man Verbindungen der allgemeinen Formel (II)

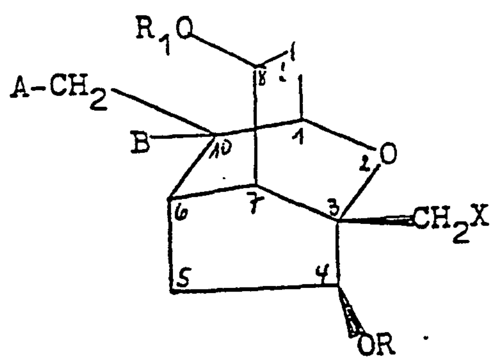

II

worin $R_1$, A und B obige Bedeutung besitzen, X Jod oder Brom bedeutet und R Wasserstoff oder niederes Acyl bedeutet, mit einer Base in Gegenwart eines Lösungsmittels behandelt.

Vorzugsweise werden Verbindungen der Formel (II) eingesetzt, worin X Jod bedeutet und R Acetyl oder Wasserstoff bedeutet.

Verbindungen der Formel (II), worin R Wasserstoff bedeutet, können durch Hydrolyse aus Verbindungen der Formel (II), worin R Acetyl bedeutet, hergestellt werden.

Als Basen eignen sich niedere Alkalimetallalkoxide, beispielsweise Natriummethylalkoholat, Alkalimetallhydroxide oder -Carbonate, beispielsweise Kaliumcarbonat oder Alkalimetallhydride wie z. B. Natriumhydrid. Ferner sind quartäre organische Ammoniumhydroxide, beispielsweise quartäre Niederalkylammoniumhydroxide, wie Tetrabutylammoniumhydroxid, oder tertiäre organische Amine geeignet. Als tertiäre organische Amine kommen insbesondere tertiäre Niederalkylamine wie Triäthylamin, Tripropylamin oder Tributylamin in Frage sowie auch cyclische tertiäre Amine wie 1,4-Dimethylpiperazin oder Pyridin.

Zweckmäßigerweise wird die Reaktion in einem Lösungsmittel, welches sowohl die Verbindung der Formel (II) wie auch die verwendete Base zu lösen vermag, durchgeführt. Als Beispiele geeigneter Lösungsmittel seien niedere Alkohole, wie z. B. Methanol oder Äthanol, offene oder cyclische Äther, wie z. B. Diäthyläther, Tetrahydrofuran oder Dioxan, oder aromatische Kohlenwasserstoffe wie Toluol oder Benzol genannt. Gegebenenfalls können diese Lösungsmittel im Gemisch mit Wasser verwendet werden. Sofern als Basen

Alkalimetallalkoholate eingesetzt werden, werden als Lösungsmittel zweckmäßigerweise die entsprechenden niederen Alkohole verwendet. Falls Hydroxide oder Carbonate eingesetzt werden, werden als Lösungsmittel vorzugsweise niedere Alkohole gegebenenfalls im Gemisch mit Wasser verwendet. Falls Alkalimetallhydride eingesetzt werden, werden als Lösungsmittel vorzugsweise cyclische Äther verwendet.

Die Reaktion kann bei Temperaturen zwischen 10°C und 110°C, vorzugsweise zwischen Raumtemperatur und 80°C, durchgeführt werden. Die Reaktionsdauer kann je nach Art des verwendeten Ausgangsproduktes und der verwendeten Reaktionsbedingungen zwischen 1 und 5 Stunden betragen. Falls R in den Verbindungen der Formel (II) eine niedere Acylgruppe bedeutet, müssen selbstverständlich die zu verwendenden Basen und deren Mengen sowie die Reaktionsbedingungen derart gewählt werden, daß sie für eine Hydrolyse der Estergruppe geeignet sind.

Es ist als überraschend anzusehen, daß nach dem erfindungsgemäßen Verfahren eine Spaltung des carbocyclischen Ringes der Verbindungen der Formel (II) unter schonenden Bedingungen ermöglicht wird. Bei der Umsetzung bleibt die Konfiguration an allen Asymmetriezentren erhalten, so daß die Stellung der Substituenten an den Zentren C5 und C8 in den Verbindungen der Formel (I) mit der entsprechenden Konfiguration der eingesetzten Ausgangsprodukte übereinstimmt.

Die erfindungsgemäßen Verbindungen der Formel (I) eignen sich als Zwischenprodukte zur Herstellung von neuen in 5-Stellung basisch substituierten 2,10-Dioxa-tricyclo-[5,3,1,$0^{3,8}$]-undecan-Derivaten mit wertvollen pharmakologischen Eigenschaften.

Beispielsweise können die Verbindungen der Formel (I) mit - Tryptamin, einem Amin der Formel (III)

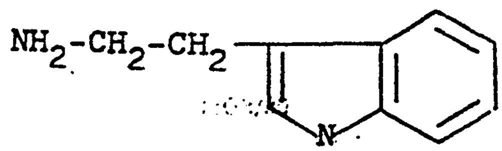

III

in einem Lösungsmittel $R_2OH$ worin $R_2$ $C_1$-$C_4$-Alkyl oder $C_1$-$C_4$-Alkanoyl bedeutet, umgesetzt werden zu Verbindungen der allgemeinen Formel (IV)

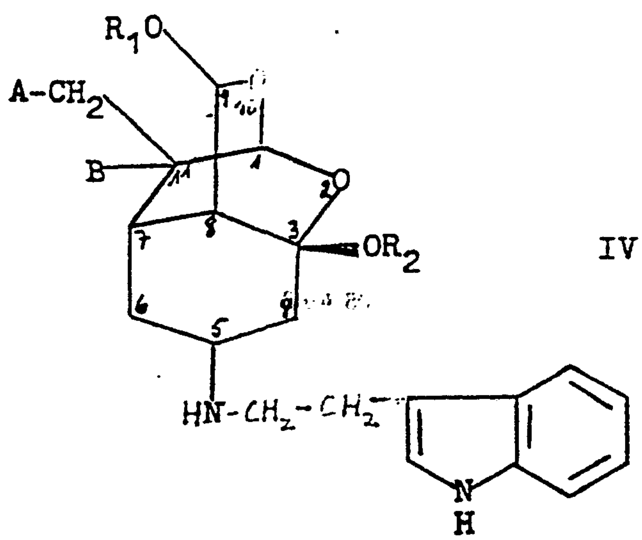

IV

worin $R_1$, $R_2$, A und B obige Bedeutung besitzen, und welche wertvolle pharmakologische Eigenschaften, insbesondere Herzkreislauf-Wirkungen, wie z. B. blutdrucksenkende Wirkungen besitzen.

So kann die Verarbeitung von Verbindungen der Formel (I) zu Verbindungen der Formel (IV), worin $R_2$, Methyl bedeutet, durch Umsetzen mit Tryptamin in Methanol beispielsweise unter den in dem nachfolgenden Beispiel A angegebenen Reaktionsbedingungen erfolgen.

Auf Grund ihrer blutdrucksenkenden Wirkung sind die Verbindungen der Formel (IV) als Antihypertensiva zur Behandlung von Bluthochdruck geeignet. Die Herzkreislauf-Wirkungen der Substanzen der Formel (IV) lassen sich in pharmakologischen Standardtestmethoden an Tieren nachweisen.

Die Wirkung der Substanzen auf Blutdruck, Herzfrequenz und EKG-Parameter bei i.v.-Dauerinfusion in narkotisierten Ratten wird nach der Methode von Buschmann et al. (J. Cardiovascular Pharmacol. 2, 777-781 (1980)) bestimmt.

Männliche Wistar-Ratten (330-370 g Körpergewicht) werden durch i.p.-Applikation von 1,25 g/kg Urethan narkotisiert und tracheotomiert. Nach einer Äquilibrierungsphase von 10 Minuten wird mit den Messungen begonnen. In einer Vorlaufphase von 5 Minuten werden die Ausgangswerte gemessen. Anschließend werden

die Prüfsubstanzen in isotonischer Natriumchloridlösung als Dauerinfusion i.v. appliziert, beginnend mit einer Dosis vom 0,01 µmol/kg/min. Die Dosis wird alle 10 Minuten ohne Erhöhung des Infusionsvolumens auf das 10-fache gesteigert. Es werden der systolische und der diastolische Blutdruck (P syst und P diast) gemessen und daraus der mittlere Blutdruck (P mittel) bestimmt, und aus dem Elektrocardiogramm (EKG) werden die Werte für die atrioventriculäre Überleitungszeit (in msec., herzfrequenz-korrigiert = PRc), das Zeitintervall der intraventriculären Erregungsausbreitung (= QRS) und die Zeitdauer vom Beginn der ventriculären Erregungsausbreitung bis zum Maximum der T-Welle (= R-αT) abgelesen. Die Herzfrequenz wird aus dem R-R-abstand bestimmt.

Aus den gemessenen Blutdruck- und Herzfrequenzwerten werden die $ED_{75}$ in µmol/kg berechnet, das ist die Gesamtdosis, welche eine Senkung dieser Parameter um 25 % ihres Ausgangswertes bewirkt. Aus den dem EKG entnommenen Werten wird die jeweilige $ED_{125}$ in µmol/kg berechnet, das ist die Gesamtdosis, welche die Parameter um 25 % ihres Ausgangswertes erhöht.

Ferner wird in dem Versuch auch die minimale letale Dosis (DL min) in µmol/kg mitbestimmt.

In der vorbeschriebenen Versuchsanordnung zeigen die Verbindungen der Formel (IV) in einem Dosisbereich von 1 bis 100 µmol/kg blutdrucksenkende Wirkungen. So wurden z. B. für das 1R,3R,5S,7R,8R,9R-N-[3,9-Dimethoxy-11-methylen-2,10-dioxa-tricyclo-[5,3,1,$0^{3,8}$]-undecan-5-yl]-tryptamin die folgenden Werte ermittelt:

| | | |
|---|---|---|
| Herzfrequenz $ED_{75}$ | 41 | µmol/kg |
| $P_{syst}$ $ED_{75}$ | 21 | µmol/kg |
| $P_{diast}$ $ED_{75}$ | 5,9 | µmol/kg |
| $P_{mittel}$ $ED_{75}$ | 9,7 | µmol/kg |
| $PR_c$ $ED_{125}$ | 93 | µmol/kg |
| QRS $ED_{125}$ | 35 | µmol/kg |
| R-αT $ED_{125}$ | 19 | µmol/kg |
| $DL_{min}$ | 210 | µmol/kg |

Aus diesen Werten ist ersichtlich, daß die Verbindung eine hohe Spezifität der blutdrucksenkenden Wirkung insbesondere auf den diastolischen Blutdruck und eine hohe Verträglichkeit aufweist.

Die Ausgangsverbindungen der Formel (II) und deren Herstellung sind bekannt, z. B. aus den DE-OS-21 29 507, 26 07 106 und 27 19 916, oder sie können nach den in diesen Offenlegungsschriften beschriebenen Verfahren bzw. analog zu den dort beschriebenen Verfahren hergestellt werden. Verbindungen der Formel (II), worin A und B je Wasserstoff bilden, können durch Hydrierung von Verbindungen der Formel (II), worin A und B gemeinsam eine Bindung bilden, hergestellt werden. Bei der Hydrierung wird ein Epimerengemisch erhalten, worin R- und S-Konfiguration im Verhältnis 9 : 1 vorliegen. Die Epimeren können durch fraktionierte Kristallisation wie in der DE-OS-27 19 916 beschrieben getrennt werden.

Die nachfolgenden Beispiele sollen die Herstellung der Verbindungen der Formel (I) näher erläutern.

**Beispiel 1**

1R, 4S, 5R, 7R-5-Methoxy-3,8-dimethylen-2,6-dioxa-bicyclo-[2,2,2]-octan-7-yl-acetaldehyd.

Eine Lösung von 7,6 g 1R,3S,4S,6R,7S,8R-3-Jodmethyl-4-acetoxy-8-methoxy-10-methylen-2,9-dioxa-tricyclo-[4,3,1,$0^{3,7}$]-decan in 100 ml absolutem Methanol wird zu einer durch Auflösen von 0,44 g Natrium in 40 ml absolutem Methanol bereiteten Natriummethylat-Lösung gegeben. Das Gemisch wird 4,5 Stunden lang bei 60°C reagieren gelassen. Anschließend wird zur Aufarbeitung auf Eiswasser gegossen, mit Natriumchlorid gut ausgesalzen und mit Äther extrahiert. Die Ätherphase wird mit einigen Tropfen Eisessig versetzt, über Natriumsulfat getrocknet, filtriert und eingeengt. Als Rückstand verbleiben 4,29 g rohes 1R,4S,5R,7R-5-Methoxy-3,8-dimethylen-2,6-dioxa-bicyclo-[2,2,2]-octan-7-yl-acetaldehyd.

Das Rohprodukt kann bereits in dieser Form als Zwischenprodukt zur weiteren Umsetzung zu einer Verbindung der Formel (IV) verwendet werden.

Zur Reindarstellung wird das Rohprodukt chromatographisch über Kieselgel unter Verwendung von n-Hexan/Äther als Elutionsmittel gereinigt. Das nach Einengen des Eluates erhaltene dünnschichtchromatographisch reine Produkt wird aus n-Hexan/Äther kristallisiert.

Fp. 56-59°C

IR-Spektrum: 3070 $cm^{-1}$, 1725 $cm^{-1}$, 1675 $cm^{-1}$, 1170 $cm^{-1}$, 1075 $cm^{-1}$, 960 $cm^{-1}$.

**Beispiel 2**

1R,4S,5R,7R-5-Methoxy-3,8-dimethylen-2,6-dioxa-bicyclo-[2,2,2]-octan-7-yl-acetaldehyd.

6 g 1R,3S,4S,6R,7S,8R-3-Jodmethyl-4-acetoxy-8-methoxy-10-methylen-2,9-dioxa-tricyclo-[4,3,1,$0^{3,7}$]-decan werden zu einer durch Auflösen von 2,5 g Natrium in 250 ml absolutem Methanol bereiteten Natriummethylat-Lösung gegeben und das Reaktionsgemisch 1,5 Stunden bei einer Badtemperatur von 60°C unter Stickstoff

gerührt. Zur Aufarbeitung wird anschließend auf ca. 1/4 des ursprünglichen Volumens am Rotationsverdampfer eingeengt und die eingeengte Lösung vorsichtig mit 100 g gesättigter $(NH_4)_2SO_4$-Lösung versetzt, wobei $NH_3$ entweicht. Anschließend wird fünfmal mit insgesamt 500 ml Äther extrahiert. Die ätherischen Extrakte werden mit 50 ml $(NH_4)_2SO_4$-Lösung und anschließend mit Wasser gewaschen, über Magnesiumsulfat getrocknet und eingedampft. Es werden 2,9 g rohes 1R,4S,5R,7R-5-Methoxy-3,8-dimethylen-2,6-dioxa-bicyclo[2,2,2]-octan-7-yl-acetaldehyd erhalten.

Dieses Rohprodukt kann ohne weitere Reinigung als Zwischenprodukt zur Herstellung von Verbindungen der Formel (IV) verwendet werden. Gewünschtenfalls kann das Produkt, wie in Beispiel 1 beschrieben, weiter gereinigt werden. Das gereinigte Produkt stimmt mit dem in Beispiel 1 erhaltenen Produkt überein.

**Beispiel 3**

1R,4S,5R,7R,8R-5-Methoxy-8-methyl-3-methylen-2,6-dioxa-bicyclo-[2,2,2]-octan-7-yl-acetaldehyd.

A. Eine Lösung von 10 g 1R,3S,4B,6R,7S,8R,10R-3-Jodmethyl-4-acetoxy-8-methoxy-10-methyl-2,9-dioxa-tricyclo-[4,3,1,$0^{3,7}$]-decan in 220 ml Methanol wird mit 3,6 g Kaliumcarbonat versetzt und die Reaktionsmischung ca. 1 Stunde bei Raumtemperatur gerührt. Zur Aufarbeitung wird das Gemisch mit Wasser verdünnt und mit Methylenchlorid extrahiert. Die organische Phase wird abgetrennt, über Natriumsulfat getrocknet, filtriert und im Vakuum zur Trockne eingeengt. Es wird 8,7 g 1R,3S,4S,6R,7R,8R,10R-3-Jodmethyl-4-hydroxy-8-methoxy-10-methyl-2,9-dioxa-tricyclo-[4,3,1,$0^{3,7}$]-decan erhalten.

Fp. 92-93°C.

B. Eine Lösung von 3,24 g 1R,3S,4S,6R,7S,8R,10R-3-Jodmethyl-4-hydroxy-8-methoxy-10-methyl-2,9-dioxa-tricyclo-[4,3,1,$0^{3,7}$]-decan in 50 ml absolutem Methanol werden zu einer durch Auflösen von 0,23 g Natrium in 50 ml absolutem Methanol gebildeten Natriummethylat-Lösung gegeben. Das Reaktionsgemisch wird ca. 3 Stunden bei einer Temperatur von 60°C gerührt. Anschließend wird zur Aufarbeitung auf Eiswasser gegossen, gut mit Natriumchlorid ausgesalzen und mit Äther extrahiert. Die Ätherphase wird mit einigen Tropfen Eisessig versetzt, über Natriumsulfat getrocknet, filtriert und im Vakuum bis zur Trockne eingeengt.

Als Rückstand werden 2,7 g rohes 1R,4S,5R,7R,8R-5-Methoxy-8-methyl-3-methylen-2,6-dioxa-bicyclo-[2,2,2]-octan-7-yl-acetaldehyd erhalten.

Dieses Rohprodukt kann ohne weitere Reinigung als Zwischenprodukt zur Herstellung von Verbindungen der Formel (IV) verwendet werden.

Zur Reindarstellung wird das Rohprodukt chromatographisch über Kieselgel unter Verwendung von n-Hexan enthaltend bis zu 15 % Äther gereinigt. Nach Einengen des Eluats wird das dünnschichtchromatographisch reine Produkt als Öl erhalten.

IR-Spektrum: 1725 $cm^{-1}$, 1675 $cm^{-1}$, 1060 $cm^{-1}$.

**Beispiel 4**

1R,4S,5R,7R,8R-5-Methoxy-8-methyl-3-methylen-2,6-dioxa-bicyclo-[2,2,2]-octan-7-yl-acetaldehyd.

1 g 1R, 3S, 4S, 6R, 7S,8R,10R-3-Jodmethyl-4-acetoxy-8-methoxy-10-methyl-2,9-dioxatricyclo-[4,3,1,$0^{3,7}$]-decan werden in 10 ml Methanol gegeben und mit 0,35 g Kaliumcarbonat versetzt. Das Reaktionsgemisch wird 5 Stunden bei 60°C gerührt. Anschließend wird zur Aufarbeitung mit Wasser verdünnt und mit Methylenchlorid extrahiert. Die organische Phase wird über Natriumsulfat getrocknet, filtriert und im Vakuum bis zur Trockne eingeengt. Es werden 0,46 g rohes 1R,4S,5R,7R,8R-5-Methoxy-8-methyl-3-methylen-2,6-dioxa-bicyclo-[2,2,2]-octan-7-yl-acetaldehyd erhalten.

Dieses Produkt kann direkt als Zwischenprodukt zur Herstellung von Verbindungen der Formel (IV) eingesetzt werden. Gewünschtenfalls kann das Produkt wie in Beispiel 3 beschrieben gereinigt werden. Das gereinigte Produkt stimmt mit dem in Beispiel 3 erhaltenen Produkt überein.

**Beispiel 5**

1R,4S,5R,7R 8R-5-Methoxy-8-methyl-3-methylen-2,6-dioxa-bicyclo-[2,2,2]-octan-7-yl-acetaldehyd.

0,14 g Natriumhydrid (50 %-ig in Öl) werden in 10 ml absolutes Tetrahydrofuran gegeben und eine Lösung von 1 g 1R,3S,4S,6R,7S,8R,10R-3-Jodmethyl-4-hydroxy-8-methoxy-10-methyl-2,9-dioxa-tricyclo-[4,3,1,$0^{3,7}$]-decan in 10 ml absolutem Tetrahydrofuran hinzugetropft, Das Reaktionsgemisch ca. 3 Stunden bei Raumtemperatur gerührt. Zur Aufarbeitung wird das Gemisch vorsichtig mit Eiswasser versetzt und anschließend mit Methylenchlorid extrahiert. Die organische Phase wird über Natriumsulfat getrocknet, filtriert und im Vakuum bis zur Trockne eingeengt. Es werden 0,68 g rohes 1R,4S,5R,7R,8R-5-Methoxy-8-methyl-3-methylen-2,6-dioxa-bicyclo-[2,2,2]-octan-7-yl-acetaldehyd erhalten.

Dieses Produkt kann direkt als Zwischenprodukt zur Herstellung von Verbindungen der Formel (IV)

eingesetzt werden. Gewünschtenfalls kann das Produkt wie in Beispiel 3 beschrieben gereinigt werden. Das gereinigte Produkt stimmt mit dem in Beispiel 3 erhaltenen Produkt überein.

**Beispiel 6**

1R,4S,5R,7R-5-Isobutoxy-3,8-dimethylen-2,6-dioxa-bicyclo-[2,2,2]-octan-7-yl-acetaldehyd.

4,5 g 1R,3S,4S,6R,7S,8R-3-Jodmethyl-4-acetoxy-8-isobutoxy-10-methylen-2,9-dioxa-tricyclo-[4,3,1,$0^{3,7}$]-decan (Fp:. 45-50° C, hergestellt auf an sich bekannte Weise durch Umsetzung von Didrovaltratum mit Isobutanol und Jodwasserstoffsäure) werden wie in Beispiel 1 beschrieben mit einer durch Auflösen von 1,25 g Natrium in 200 ml Methanol bereiteten Natriummethylat-Lösung umgesetzt. Nach Aufarbeitung des Reaktionsgemisches werden 3,5 g rohes 1R,4S,5R,7R-5-Isobutoxy-3,8-dimethylen-2,6-dioxa-bicyclo-[2,2,2]-octan-7-yl-acetaldehyd als Öl erhalten.

IR-Spektrum: 3070 $cm^{-1}$, 1725 $cm^{-1}$, 1675 $cm^{-1}$, 1175 $cm^{-1}$, 1075 $cm^{-1}$, 960 $cm^{-1}$.

Dieses Produkt kann direkt als Zwischenprodukt zur Herstellung von Verbindungen der Formel (IV) eingesetzt werden.

**Beispiel 7**

1R,4S,5R,7R-5-Benzyloxy-3,8-dimethylen-2,6-dioxa-bicyclo-[2,2,2]-octan-7-yl-acetaldehyd.

4,2 g 1R,3S,4S,6R,7S,8R-3-Jodmethyl-4-acetoxy-8-benzyloxy-10-methylen-2,9-dioxa-tricyclo-[4,3,1,$0^{3,7}$]-decan (Fp.: 69-70° C, hergestellt auf an sich bekannte Weise durch Umsetzung von Didrovaltratum mit Benzylalkohol und Jodwasserstoffsäure) werden mit einer durch Auflösen von 1,2 g Natrium in 200 ml Methanol hergestellten Natriummethylat-Lösung wie in Beispiel 1 beschrieben umgesetzt. Nach Aufarbeitung des Reaktionsgemisches werden 3,2 g rohes 1R,4S,5R,7R-5-Benzyloxy-3,8-dimethylen-2,6-dioxa-bicyclo-[2,2,2]-octan-yl-acetaldehyd als Öl erhalten.

IR-Spektrum: 3065 $cm^{-1}$, 3030 $cm^{-1}$, 1725 $cm^{-1}$, 1675 $cm^{-1}$, 1495 $cm^{-1}$, 1070 $cm^{-1}$, 960 $cm^{-1}$.

Dieses Produkt kann direkt als Zwischenprodukt zur Herstellung von Verbindungen der Formel (IV) eingesetzt werden.

**Beispiel A**

Weiterverarbeitung von 1R,4S,5R,7R-5-Methoxy-3,8-dimethylen-2,6-dioxa-bicyclo-[2,2,2]-octan-7-yl-acetaldehyd zu 1R,3R,5S,7R,8R,9R- und 1R,3R,5R,7R,8R,9R-N-(3,9-Dimethoxy-11-methylen-2,10-dioxatricyclo-[5,3,1,$0^{3,8}$]-undecan-5-yl)-tryptamin.

Aus 4 g 1R,3S,4S,6R,7S,8R-3-Jodmethyl-4-acetoxy-8-methoxy-10-methylen-2,9-dioxa-tricyclo-[4,3,1,$0^{3,7}$]-decan nach der Vorschrift des Beispiels 2 erhaltenes rohes 1R,4S,5R,7R-5-Methoxy-3,8-dimethylen-2,6-dioxa-bicyclo-[2,2,2] octan-7-yl-acetaldehyd werden in 100 ml absolutem Methanol gelöst und die Lösung unter Stickstoff mit 2 g Tryptamin-hydrochlorid versetzt und das Reaktionsgemisch bei einer Badtemperatur von 60° C 3 1/2 Stunden gerührt. Anschließend wird zur Aufarbeitung das Methanol abdestilliert, das verbleibende Reaktionsgemisch mit konzentrierter Sodalösung alkalisiert und mit Methylenchlorid extrahiert. Die organische Phase wird über Magnesiumsulfat getrocknet und eingedampft. Es werden 4 g rötlich gefärbtes Rohprodukt erhalten. Dieses wird durch Säulenchromatographie unter Druck unter Verwendung von Chloroform enthaltend 5 % Methanol als Elutionsmittel gereinigt. Aus dem Eluat werden 890 mg unpolares 1R,3R,5S,7R,8R,9R-N-(3,9-Dimethoxy-11-methylen-2,10-dioxa-tricyclo-[5,3,1,$0^{3,8}$]-undecan-5-yl)-tryptamin als farbloses Schaumharz erhalten.

$[\alpha]^{20}_{D} = + 3,3°$

Molekulargewicht: berechnet 384,2049; gefunden 384,2048

Massenspektrum (130° C): M+ = 384(4), 383(11), 352(11), 253(43), 221(21), 192(27), 179(42), 175(9), 144(11), 131(100).

Ferner wird aus dem Eluat als polares Nebenprodukt rohes 1R,3R,5R,7R,8R,9R-N-(3,9-Dimethoxy-11-methylen-2,10-dioxa-tricyclo-[5,3,1,$0^{3,8}$]-undecan-5-yl)-tryptamin erhalten. Nach nochmaliger Reinigung durch präparative Dünnschichtchromatographie unter Verwendung von Chloroform enthaltend 5 % Methanol als Elutionsmittel werden 125 mg reines Produkt als farbloses Schaumharz erhalten.

Molekulargewicht: berechnet 384,2049; gefunden 384,2048

Massenspektrum (110° C): M+ = 384(7) 353(8), 254(100), 242(12).

**Patentansprüche** für die Vertragsstaaten BE, CH, DE, FR, GB, NL, IT, SE.

1. Verbindungen der allgemeinen Formel (I)

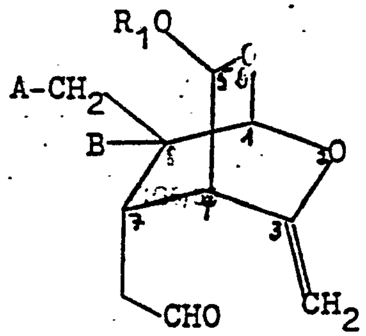

I

worin $R^1$, Alkyl mit 1-4 Kohlenstoffatomen oder Benzyl bedeutet und A und B je Wasserstoff bedeuten oder gemeinsam eine Bindung bilden.

2. Verfahren zur Herstellung von Verbindungen der allgemeinen Formel (I)

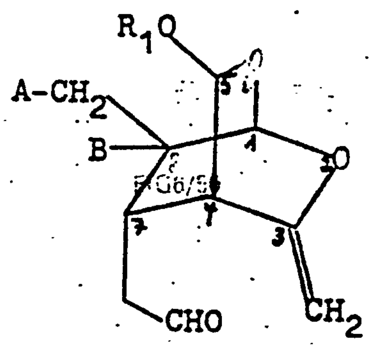

I

worin $R_1$, A und B die in Anspruch 1 angegebene Bedeutung besitzen, dadurch gekennzeichnet, daß man Verbindungen der allgemeinen Formel (II)

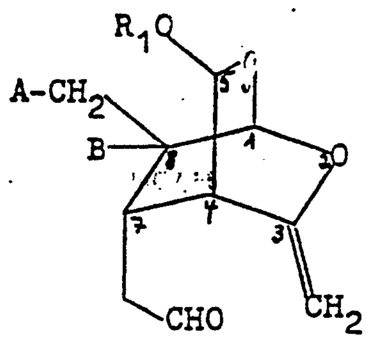

I

worin $R_1$, A und B obige Bedeutung besitzen, X Jod oder Brom bedeutet und R Wasserstoff oder niederes Acyl bedeutet, mit einer Base in Gegenwart eines Lösungsmittels behandelt.

**Patentanspruch** für den Vertragsstaat AT

Verfahren zur Herstellung von Verbindungen der allgemeinen Formel (I)

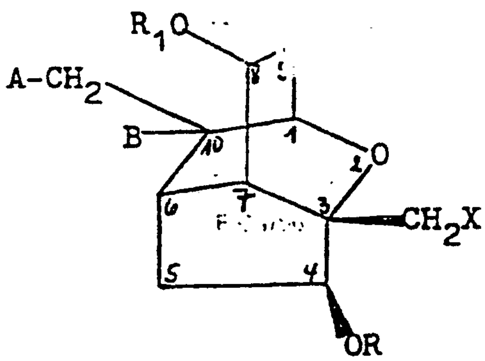

II

worin $R_1$ Alkyl mit 1-4 Kohlenstoffatomen oder Benzyl bedeutet und A und B je Wasserstoff bedeuten oder gemeinsam eine Bindung bilden, dadurch gekennzeichnet, daß man Verbindungen der allgemeinen Formel (II)

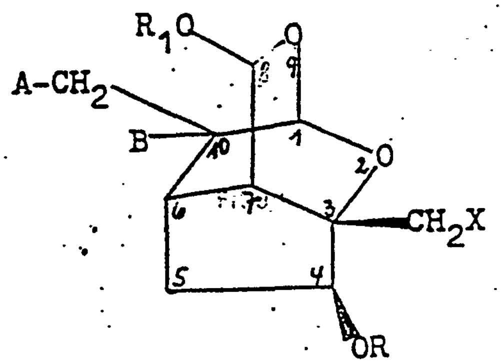

II

worin $R^1$, A und B obige Bedeutung besitzen, X Jod oder Brom bedeutet und R Wasserstoff oder niederes Acyl bedeutet, mit einer Base in Gegenwart eines Lösungsmittels behandelt.

**Claims** for the Contracting States BE, CH, DE, FR, GB, NL, IT, SE.

1. Compounds of the general Formula (I)

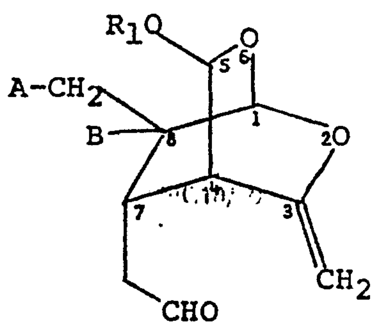

I

in which $R_1$ is alkyl with 1 to 4 carbon atoms or benzyl and A and B are each hydrogen or together form a bond.

2. Method for the preparation of compounds of the general Formula (I)

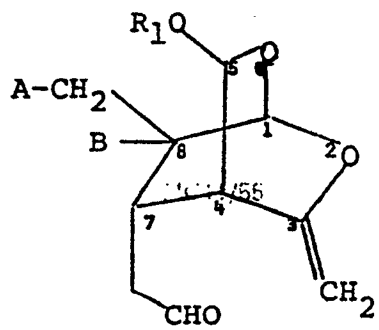

I

in which R1, A and B have the meanings indicated in Claim 1, characterised in that compounds of the general Formula (II)

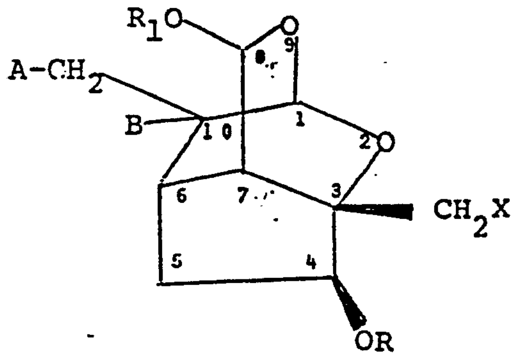

II

in which $R_1$, A and B have the above meanings, X is iodine or bromine and R is hydrogen or lower acyl, are treated with a base in the presence of a solvent.

**Claim** for the Contracting State AT

1. Method for the preparation of compounds of the general Formula (I)

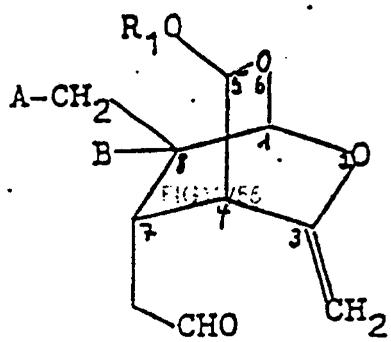

I

in which $R_1$ is alkyl with 1 to 4 carbon atoms or benzyl and A and B are each hydrogen or together form a bond, characterised in that compounds of the general Formula (II)

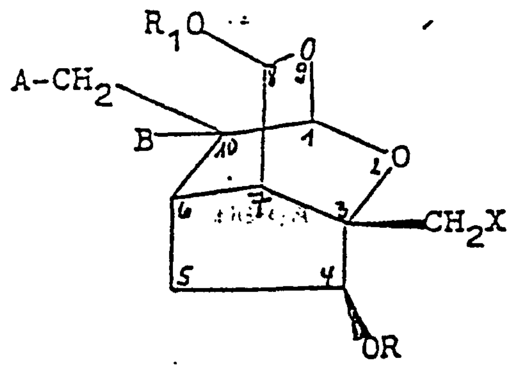

II

in which $R_1$, A and B have the above meanings, X is iodine or bromine and R is hydrogen or lower acyl, are treated with a base in the presence of a solvent.

**Revendications** pour les Etats contractants BE, CH, DE, FR, GB, IT, NL, SE.

1. Composés de formule générale (I)

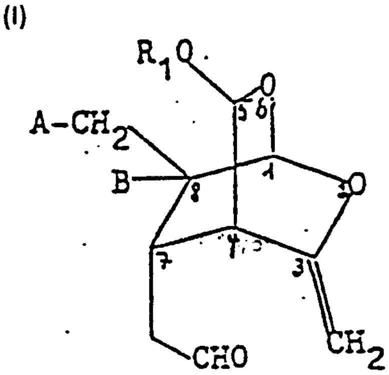

I

dans laquelle $R_1$ représente un groupe alkyle ayant 1 à 4 atomes de carbone ou un groupe benzyle, et A et B représentent chacun un atome d'hydrogène ou forment ensemble une liaison.

2. Procédé pour préparer des composés de formule générale (I)

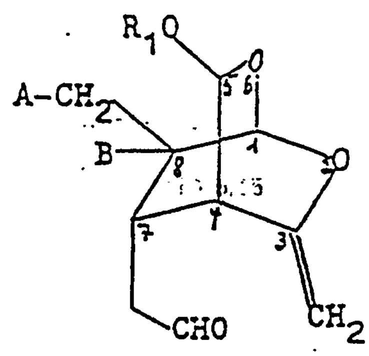

I

dans laquelle $R_1$, A et B ont le sens indiqué à la revendication 1, caractérisé en ce qu'on traite par une base, en présence d'un solvant, des composés de formule générale (II)

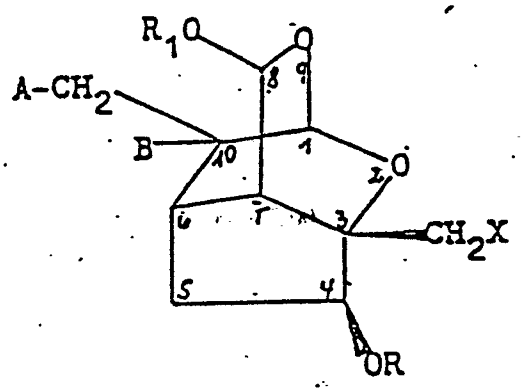

II

dans laquelle A et B ont le sens ci-dessus, X représente de l'iode ou du brome et R représente un atome d'hydrogène ou un groupe acyle inférieur.

**Revendication** pour l'Etat contractant AT

Procédé pour préparer des composés de formule générale (I)

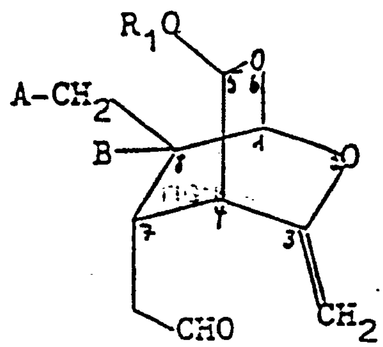

I

dans laquelle $R_1$ représente un groupe alkyle ayant 1 à 4 atomes de carbone ou un groupe benzyle, et A et B représentent chacun un atome d'hydrogène ou forment ensemble une liaison, caractérisé en ce qu'on traite par une base, en présence d'un solvant, des composés de formule générale (II):

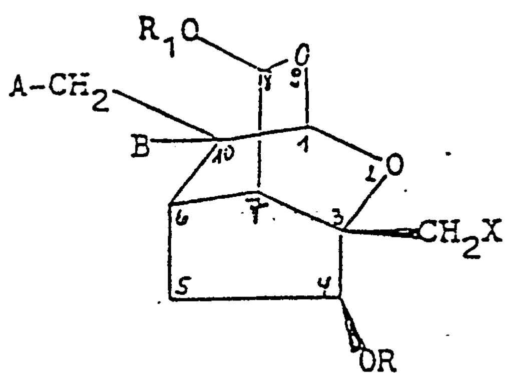

II

dans laquelle $R_1$, A et B ont le sens précité, X représente de l'iode ou du brome et R représente un atome d'hydrogène ou un groupe acyle inférieur.